# EUROPEAN PATENT APPLICATION

(11) **EP 3 412 777 A1**
(43) Date of publication of application: **12.12.2018**
(21) Application number: 17747499.6
(22) Date of filing: 02.02.2017
(51) Int. Cl.: C12Q 1/68, C12N 15/09, G01N 27/62, G01N 30/88, G01N 33/53

(54) **METHOD FOR MEASURING TARGET DNA**

(30) Priority: 03.02.2016 JP 2016019137
(71) Applicant: Miraca Research Institute G.K., Tokyo 192-0031 (JP)
(72) Inventor: MATSUNO, Tatsuki, Tokyo 163-0410 (JP); ABE, Koichi, Tokyo 163-0410 (JP)
(74) Representative: Hoffmann Eitle
(86) International application number: PCT/JP2017/003707
(87) International publication number: WO 2017/135348

(57) **Abstract**

The present invention provides a method for adequately analyzing a target DNA after genomic DNA cleavage reaction with a restriction enzyme. More specifically, the present invention provides a method for measuring a target DNA, including
(1) cleaving a genomic DNA with a restriction enzyme in a solution to obtain a mixed solution containing (a) completely cleaved DNA fragments consisting of the target DNA, (b) incompletely cleaved DNA fragments comprising the target DNA, and (c) DNA fragments not comprising the target DNA;
(2) removing the DNA fragments of (b) from said mixed solution to obtain a solution containing the DNA fragment of (a) and the DNA fragments of (c); and
(3) analyzing the target DNA in the solution obtained in (2) .

SEQUENCE LISTING

<110>  FUJIREBIO INC.

<120>  Method for specifically detecting target nucleic acid

<130>  PRBA-281698

<150>  JP 2016-019137
<151>  2016-02-03

<160>  6

<170>  PatentIn version 3.5

<210>  1
<211>  18
<212>  DNA
<213>  Artificial Sequence

<220>
<223>  Forward Primer

<400>  1
tctagacccc gccccacg                                                    18


<210>  2
<211>  21
<212>  DNA
<213>  Artificial Sequence

<220>
<223>  Reverse Primer

<400>  2
ctgcaggacc actcgaggct g                                                21


<210>  3
<211>  24
<212>  RNA
<213>  Artificial Sequence

<220>
<223>  Nucleotide sequence of nucleic acid probe

<400>  3
ugcaggacca cucgaggcug ccac                                             24


<210>  4
<211>  21
<212>  DNA
<213>  Artificial Sequence

<220>
<223>  Forward Primer

<400>  4
tagaacgctt tgcgtcccga c                                                21

<210>  5
<211>  19
<212>  DNA
<213>  Artificial Sequence

<220>
<223>  Reverse Primer

<400>  5
gagagctccg cactcttcc                                                    19


<210>  6
<211>  48
<212>  DNA
<213>  Artificial Sequence

<220>
<223>  Nucleotide sequence of nucleic acid probe

<400>  6
gtggggcggg gtctagagaa aaaaaaaaaa aaaaaaaaaa aaaaaaaa                    48

## Description

### TECHNICAL FIELD

The present invention relates to a method for measuring a target DNA.

### BACKGROUND ART

Prior to analysis of target nucleic acids, a purity or a concentration of the target nucleic acids is often increased. For example, Patent Literature 1 discloses that non-target nucleic acids are removed from a mixed solution containing a target nucleic acid and the non-target nucleic acids. Patent Literature 2 discloses that a target nucleic acid is isolated or purified from a mixed solution containing the target nucleic acid and non-target nucleic acids.

### PRIOR ART REFERENCES

### PATENT LITERARURES

Patent Literature 1: WO2010/091870
Patent Literature 2: WO2006/121888

### SUMMARY OF INVENTION

### PROBLEM TO BE SOLVED BY THE INVENTION

The present inventors have found that there is a problem that possibly a target DNA cannot adequately be analyzed when a genomic DNA is not cleaved completely in analysis of the target DNA that requires a genomic DNA cleavage reaction with a restriction enzyme.

For example, when a modified nucleobase present in the target DNA is detected after the genomic DNA cleavage reaction with the restriction enzyme, not only the modified nucleobase present in the target DNA but also a modified nucleobase present in non-target DNA linked to the target DNA through one or more non-cleavage sites on a 5'- or 3'-terminal side of the target DNA in the genomic DNA are possibly detected.

Also, when at least a partial region of the non-target DNA can be paired with the target DNA, the non-target DNA can efficiently be bound to the target DNA in a complementary manner. Thus, this possibly poses an obstacle to analysis of the target DNA using a nucleic acid probe.

That is, it is an object of the present invention to provide a method for being able to adequately analyze a target DNA after a genomic DNA cleavage reaction with a restriction enzyme.

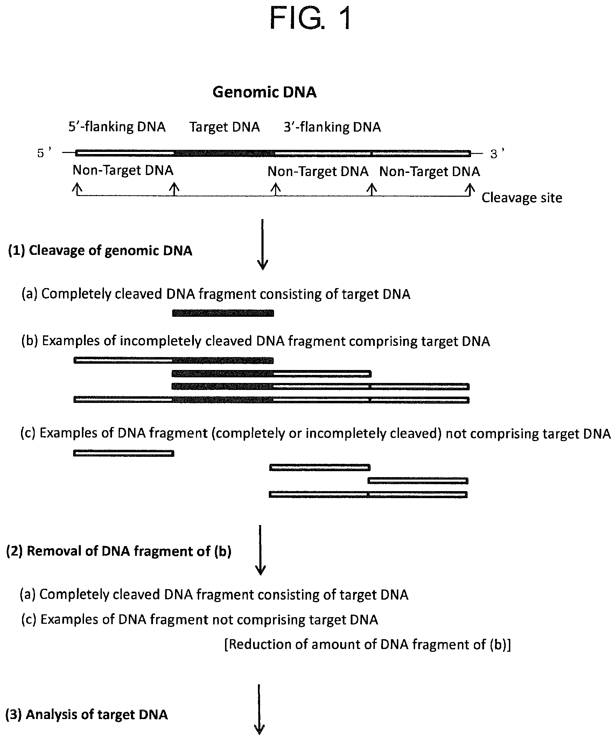

As a result of an extensive study, the present inventors have conceived that a target DNA should be analyzed after removing incompletely cleaved DNA fragments comprising the target DNA when the target DNA is analyzed after a genomic DNA cleavage reaction with a restriction enzyme (e.g., see Fig. 1), and have arrived at completing the present invention. Although patent Literatures 1 and 2 disclose that a purity and a concentration of a target nucleic acid are increased, they neither disclose nor suggest that incompletely cleaved DNA fragments comprising the target DNA are removed and that the target DNA is analyzed after removing the incompletely cleaved DNA fragment comprising the target DNA.

That is, the present invention is as follows;
[1] A method for measuring a target DNA, including
   (1) cleaving a genomic DNA with a restriction enzyme in a solution to obtain a mixed solution containing (a) a completely cleaved DNA fragment consisting of the target DNA, (b) incompletely cleaved DNA fragments comprising the target DNA, and (c) DNA fragments not comprising the target DNA;
   (2) removing (b) the incompletely cleaved DNA fragments comprising the target DNA from said mixed solution to obtain a solution containing (a) the completely cleaved DNA fragment consisting of the target DNA and (c) the DNA fragments not comprising the target DNA; and
   (3) analyzing the target DNA in the solution obtained in (2) ;
[2] The method of [1], wherein said removal is performed using a nucleic acid probe specific for non-target DNA.
[3] The method of [2], wherein the nucleic acid probe specific for the non-target DNA is a following nucleic acid probe:
   (1) a nucleic probe specific for 5'-flanking DNA;
   (2) a nucleic probe specific for 3'-flanking DNA; or
   (3) a combination of the nucleic probe specific for the 5'-flanking DNA and the nucleic probe specific for the 3'-flanking DNA.
[4] The method of any of [1] to [3], wherein said analysis is performed using a nucleic probe specific for the target DNA.
[5] The method of any of [1] to [4], wherein a modified nucleobase in the target DNA is analyzed.
[6] The method of [5], wherein the modified nucleobase is methylcytosine.
[7] The method of any of [1] to [6], wherein said analysis is performed by immunoassay, mass spectrometry, electrochemical analysis, high performance liquid chromatography analysis, or nanopore analysis.

### EFFECT OF THE INVENTION

The method of the present invention can adequately analyze the target DNA after the genomic DNA cleavage reaction with the restriction enzyme. The method of the present invention is particularly useful when a restriction enzyme with low cleavage efficiency is used for cleaving a genomic DNA. The method of the present invention is also particularly useful when a period of time for the genomic DNA cleavage reaction with the restriction enzyme is needed to be shortened because of shortening of total time required for measuring the target DNA.

### BRIEF DESCRIPTION OF DRAWINGS

Fig. 1 shows an outline of the method of the present invention.
Fig. 2 shows amounts of uncleaved DNA after a genomic DNA cleavage reaction with a restriction enzyme for various reaction times.
Fig. 3 shows amounts of uncleaved DNA captured by a nucleic acid probe specific for a target DNA.
Fig. 4 shows removal efficiency of incompletely cleaved DNA fragments comprising a target DNA using a nucleic acid probe specific for non-target DNA (relative evaluation of uncleaved rate).

### EMBODIMENTS FOR CARRYING OUT THE INVENTION

The method of the present invention is performed by the following (1) to (3):
(1) cleaving a genomic DNA with a restriction enzyme in a solution to obtain a mixed solution containing (a) a completely cleaved DNA fragment consisting of a target DNA, (b) incompletely cleaved DNA fragments comprising the target DNA, and (c) DNA fragments not comprising the target DNA;
(2) removing (b) the incompletely cleaved DNA fragments comprising the target DNA from said mixed solution to obtain a solution comprising (a) the completely cleaved DNA fragment consisting of the target DNA and (c) the DNA fragments not comprising the target DNA; and
(3) analyzing the target DNA in the solution obtained in (2) .

### (1) Cleavage of genomic DNA

A genomic DNA used in the present invention is obtained from any biological sample. Examples of organisms from which such a biological sample is derived include animals such as mammalian animals (e.g., humans, monkeys, mice, rats, rabbits, cattle, swine, horses, goats, sheep) and birds (e.g., chickens), insects, microorganisms, plants, fungi, fishes, and viruses. The biological sample may also be blood related samples that are blood itself and samples derived from the blood (e.g., whole blood, serum, plasma), saliva, urine, milk, tissues or cell extracts, or mixtures thereof. The biological samples may further be those derived from mammalian animals affected with a disease (e.g., cancer, leukemia), or mammalian animals potentially suffering from the disease. The genomic DNA can appropriately be obtained from such a biological sample by any method (e.g., genomic DNA extraction method).

By cleaving the genomic DNA with a restriction enzyme in a solution, a mixed solution is obtained which contains the following DNA fragments (see, e.g., Fig. 1):
(a) a completely cleaved DNA fragment consisting of a target DNA
(b) incompletely cleaved DNA fragments comprising the target DNA, and
(c) various DNA fragments not comprising the target DNA.

Cleavage of the genomic DNA with the restriction enzyme can be performed at appropriate temperature and for an appropriate period of time depending on a type of the restriction enzyme. The restriction enzymes used in the present invention may be those that are enzymes having an ability to cleave double stranded DNA and may produce either a blunt-end or a cohesive-end by cleavage of the double stranded DNA. Examples of such restriction enzymes include AatII, AccI, AccII, AccIII, AclI, AfaI, AflII, AluI, Aor13HI, Aor51HI, ApaI, ApaLI, AsuII, BalI, BamHI, BanII, BciT130I, BcnI, BglI, BglII, BlnI, BmeT110I, BmgT120I, Bpu1102I, Bsp1286I, Bsp1407I, BspT104I, BspT107I, BssHII, Bst1107I, BstPI, BstXI, Cfr10I, ClaI, CpoI, DdeI, DraI, DpnI, EaeI, Eam1105I, Eco52I, Eco81I, Eco065I, Eco0109I, EcoRI, EcoRII, EcoRV, EcoT14, EcoT22I, FbaI, FokI, HaeII, HaeIII, HapII, HhaI, Hin1I, HincII, HindIII, HinfI, HpaI, KpnI, MboI, MboII, MflI, MluI, MspI, MunI, NaeI, NcoI, NdeI, NheI, NotI, NruI, NsbI, PmaCI, PshAI, PshBI, Psp1406I, PstI, PvuI, PvuII, RspRSII, SacI, SacII, SalI, Sau3AI, ScaI, SfiI, SmaI, SmiI, SnaBI, SpeI, SphI, Sse8387I, SspI, StuI, TaqI, Tth111I, Van91I, VpaK11BI, XbaI, XhoI, and XspI. A period of time for the genomic DNA cleavage reaction with the restriction enzyme may be shortened because of shortening of total time required for measuring the target DNA. It is because the method of the present invention can adequately analyze the target DNA in the genomic DNA even when the cleavage of the genomic DNA with the restriction enzyme is incomplete.

### (2) Removal of incompletely cleaved DNA fragments comprising target DNA

Incompletely cleaved DNA fragments comprising the target DNA can be removed by utilizing non-target DNA in the incompletely cleaved DNA fragments. For example, such removal can be performed by removal of the incompletely cleaved DNA fragments using nucleic acid probes specific for the non-target DNA or by removal of the incompletely cleaved DNA fragments by fractionation based on a sum of molecular weights of the target DNA and the non-target DNA.

Preferably, the removal can be performed using the nucleic acid probes specific for non-target DNA. The nucleic acid probe specific for the non-target DNA means a nucleic acid probe that is complementary to a part of or the full-length of the non-target DNA linked to the target DNA via one or more (for example, 1 to 3, preferably 1 or 2, more preferably 1) restriction enzyme recognition sites (non-cleavage sites) present on a 5'- or 3'-terminal side of the target DNA in the genomic DNA (e.g., see Fig. 1).

The nucleic acid probe specific for the non-target DNA may be a DNA probe or a heterogeneous nucleic acid probe.

The heterogeneous nucleic acid probe means a nucleic acid probe having a backbone structure different from a backbone structure of the target DNA (structure composed of a sugar moiety and a phosphate moiety) as a part or a whole of the backbone structure. Examples of the heterogeneous nucleic acid probe include RNA probes (e.g., normal RNA probe composed of natural ribonucleotides having a hydroxyl group at position 2' and modified RNA probe composed of ribonucleotides where the hydroxyl group at position 2' is modified with an alkyl group such as a methyl group), peptide nucleic acid (PNA) probes, locked nucleic acid (LNA) probes, or bridged nucleic acid (BNA) probes, phosphorothioate (phosphorothioated) nucleic acid probes, as well as chimera type nucleic acid probes where such two or more nucleic acid probes are linked, and chimera type nucleic acid probes where such one or more nucleic acid probes and a DNA probe are linked.

In light of low cost, the nucleic acid probe specific for the non-target DNA may be the DNA probe.

The number of nucleotide residues that composes a nucleic acid probe specific for non-target DNA (i.e., length of the nucleic acid probe specific for the non-target DNA) is not particularly limited as long as it has a length long enough to be capable of hybridizing to the non-target DNA, and may be, for example, 12 or more, preferably 15 or more, preferably 18 or more, and more preferably 20 or more. The number of the nucleotides that composes the nucleic acid probe specific for the non-target DNA may also be, for example, 100 or less, 80 or less, 60 or less, or 50 or less. The nucleic acid probe specific for the non-target DNA can be prepared by a probe synthesis method known in the art.

The nucleic acid probe specific for the non-target DNA can be used in a free form or a form immobilized to a solid phase. The nucleic acid probe specific for the non-target DNA may be labeled with a substance or a group that allows for immobilizing to the solid phase. For example, a 5'-terminus or a 3'-terminus of the nucleic acid probe specific for the non-target DNA can be labeled. Examples of the group or the substance that allows for immobilizing to the solid phase include groups or substances that allow for covalently binging to the solid phase, or affinity substances. Examples of the group or the substance that allows for covalently binging to the solid phase include a thiol group or a substance having a thiol group (such a thiol group introduced into the nucleic acid probe specific for the non-target DNA can be bound to a maleimide group on the solid phase), an amino group or a substance having an amino group (such an amino group introduced into the nucleic acid probe specific for the non-target DNA can be bound to maleic anhydride on the solid phase). Examples of the affinity substance include streptavidin, biotin, digoxigein, dinitrophenol, fluorescein, fluorescein isothiocyanate, and a pair of mutually complementary single stranded nucleic acids having an ability to form a double stranded nucleic acid (e.g., a pair of a single stranded nucleic acid having a poly A sequence and a single stranded nucleic acid having a poly T sequence). In this case, those coated with another affinity substance that has the affinity to an affinity substance that the nucleic acid probe specific for the non-target DNA has can be used as the solid phase. When used in a free form, the nucleic acid probe specific for the non-target DNA may be immobilized to the solid phase after hybridizing with an incompletely cleaved DNA fragment comprising the target DNA.

Examples of the solid phase include particles (e.g., magnetic particles); supports such as membranes (e.g., nitrocellulose membranes), glasses, plastics and metals; and containers such as plates (e.g., multiwell plates) and tubes.

More preferably, the nucleic acid probe specific for the non-target DNA is as follows:
(i) a nucleic acid probe specific for 5'-flanking DNA;
(ii) a nucleic acid probe specific for 3'-flanking DNA;
(iii) a combination of the nucleic acid probe specific for the 5'-flanking DNA and the nucleic acid probe specific for the 3'-flanking DNA.

The 5'-flanking DNA means non-target DNA (restriction enzyme cleavage unit) adjacent to target DNA via a restriction enzyme recognition site (non-cleavage site) present on a 5'-terminal side of the target DNA in the genomic DNA (e.g., see Fig. 1). Therefore, the nucleic acid probe specific for the 5'-flanking DNA means a nucleic acid probe that is complementary to a part of or the full-length of such 5'-flanking DNA.

The 3'-flanking DNA means non-target DNA (restriction enzyme cleavage unit) adjacent to target DNA via a restriction enzyme recognition site (non-cleavage site) present on a 3'-terminal side of the target DNA in the genomic DNA (e.g., see Fig. 1). Therefore, the nucleic acid probe specific for the 3'-flanking DNA means a nucleic acid probe that is complementary to a part of or the full-length of such 3'-flanking DNA.

Incompletely cleaved DNA fragments comprising the target DNA can efficiently be removed using such 5'-flanking DNA and/or 3'-flanking DNA.

The 5'-flanking DNA and the 3'-flanking DNA are DNA probes or heterogeneous nucleic acid probes, and may be the DNA probes in light of low cost.

In more details, step (2) may be performed as follows:
(2-1) combining the nucleic acid probe specific for the non-target DNA with the mixed solution;
(2-2) incubating the mixed solution to obtain a hybridization complex containing the above nucleic acid probe and the DNA fragment of (b);
(2-3) separating the hybridization complex from or in the mixed solution to obtain the solution containing the DNA fragments of (a) and (c).

In step (2-1), the above nucleic acid probe in an appropriate amount is combined with the mixed solution. For example, the above nucleic acid probe may be added to the mixed solution. Alternatively, the mixed solution may be transferred to the solid phase to which the above nucleic acid probe is immobilized. In order to efficiently remove the incompletely cleaved DNA fragments comprising the target DNA, the above nucleic acid probe may be used in an excessive amount based on its target site (one equivalent) in the genomic DNA.

In step (2-2), the hybridization complex is obtained by incubating the mixed solution. The incubation can be performed under any condition where the hybridization complex can be formed. Such conditions (e.g., salt concentration in the solution, incubation temperature, incubation period) are well-known (see, e.g., WO2010/091870, WO2006/121888, WO2015/025862, WO2015/025863, WO2015/025864, WO2015/108177).

In step (2-3), the separation can be performed, for example, by utilizing a solid phase. When the above nucleic acid probe is immobilized to magnetic particles as the solid phase, the magnetic particles in the mixed solution can be collected by magnetic manipulation. Thus, an objective solution comprising the above DNA fragments (a) and (c), in which an amount of the above DNA fragment of (b) above is reduced, can be obtained by collecting a mixed solution supernatant containing no magnetic particle. When the above nucleic acid probe is immobilized to a support or a container as the solid phase, an objective solution can be obtained by transferring the mixed solution from the support or the container. Even if the above nucleic acid probe is not immobilized to the solid phase, when it is labeled with a substance or a group that allows for immobilizing to the solid phase, it is possible to immobilize to the solid phase after forming a hybridization complex. Thus, an objective solution can be obtained as similar to the above situation.

### (3) Analysis of target DNA

Target DNA can be analyzed by any methods (e.g., WO2015/025862; WO2015/025863; WO2015/025864; WO2015/108177; DNA Research 13, 37-42 (2006); Analytical Chemistry 77(2), 504-510 (2005); Analytical Chemistry 83, 7595-7599 (2011); Nucleic Acids Research 34(8), e61(2006); Scientific Reports 501(2), srep00501 (2012)). For example, a target DNA may be analyzed after capturing the target DNA using a nucleic acid probe specific for the target DNA.

A nucleic acid probe specific for a target DNA means a nucleic acid probe that is complementary to a part of or the full-length of the target DNA (an unit cleavage with a restriction enzyme, see Fig. 1) found in the genomic DNA. The nucleic acid probe specific for the target DNA is a DNA probe or a heterogeneous nucleic acid probe as described above, and may be the heterogeneous nucleic acid probe in light of high specificity or the like.

The number of nucleotide residues that composes the nucleic acid probe specific for the target DNA is the same as those described for the nucleic acid probe specific for the non-target DNA.

The nucleic acid probe for the target DNA can be used in a free form or in a form immobilized to a solid phase. The nucleic acid probe for the target DNA may be labeled with a substance or a group that allows for immobilizing to the solid phase. Details for the label, the solid phase, the group that allows for immobilizing to the solid phase and the substance that allows for immobilizing to the solid phase are the same as those described for the nucleic acid probe specific for the non-target DNA.

In the method of the present invention, a particular region in the target DNA, for example, modified nucleobases in that region may be analyzed. The modified nucleobase refers to a nucleobase having a modified structure in a nucleobase. Examples of the term "nucleobase" include adenine (A), guanine (G), cytosine (C) and thymine (T). The nucleobase is preferably cytosine (C). Examples of the modification include introduction of a substituent into a nucleobase, leaving a group (e.g., amino, oxo, and methyl groups) that a nucleobase has, and exchange of a group that a nucleobase has to a substituent. The substituent is not particularly limited as long as it can be possessed by a naturally occurring nucleobase, and examples of the substituent include substituents possessed by modified nucleobases in modified nucleotides described in Administrative Instructions under the Patent Cooperation Treaty (version implemented on January 1, 2009), Annex C, Appendix 2, Table 2: List of Modified Nucleotides. The nucleobases described in this document can be identical to the nucleobases described in Table 2. List of Modified Bases in Appendix 2 in "Guideline for Preparation of a Specification, etc. including Nucleotide Sequences or Amino Acid Sequences (January in 2002 or December in 2009)" published by the Japanese Patent Office. Therefore, the above Guideline can also be referenced for the modified nucleobases. The substituents are preferably methyl, hydroxymethyl and carboxyl, more preferably methyl and hydroxymethyl, and still more preferably methyl. A position to be modified in substitution and the like is not particularly limited and examples of the position are position 2, positions 4 to 6 (preferably the position is position 5) in the case of nucleobases having a pyrimidine ring (C or T), and positions 2, 6 and 8 in the case of nucleobases having a purine ring (A or G).

The modified nucleobase is not particularly limited as long as it is naturally occurring one, and examples of the modified nucleobase include modified nucleobases possessed by modified nucleotides described in Administrative Instructions under the Patent Cooperation Treaty (version implemented on January 1, 2009), Annex C, Appendix 2, Table 2: List of Modified Nucleotides. The modified nucleotides described in this document can be identical to the modified nucleotides described in Appendix 2, Table 2: List of Modified Bases in the above Guideline. Therefore, the above Guideline can also be referenced for the modified nucleobases. The modified nucleobase is preferably methylcytosine (e.g., 5-methylcytosine), hydroxymethylcytosine (e.g., 5-hydroxymethylcytosine) or carboxylcytosine (5-carboxylcytosine), more preferably methylcytosine or hydroxymethylcytosine, and still more preferably methylcytosine. The modified nucleobase is known to bring about a functional change of a nucleic acid (e.g., change of transcription regulation ability in a given gene).

The modified nucleobase as described above can be analyzed by any methods known in the art. For example, the modified nucleobase can be analyzed by immunoassay (e.g., WO2015/025862; WO2015/025863; WO2015/025864; WO2015/108177; DNA Research 13, 37-42 (2006)), mass spectrometry (e.g., Analytical Chemistry 77(2), 504-510 (2005)), electrochemical analysis (e.g., Analytical Chemistry 83, 7595-7599 (2011)), high performance liquid chromatography analysis (e.g., Nucleic Acids Research 34(8), e61 (2006)) or nanopore analysis (e.g., Scientific Reports 501(2), srep00501 (2012)). For example, the analysis may be performed after capturing the target DNA to the solid phase. For example, when the modified nucleobases are analyzed by mass spectrometry, electrochemical analysis or high performance liquid chromatography analysis, the modified nucleobases can be analyzed by decomposing the target DNA captured on the solid phase with endonuclease or/and exonuclease, recovering a solution in which DNA fragments are decomposed into monomer bases (nucleotides), and subjecting this solution to such technology. Also, when the modified nucleobases are analyzed by the nanopore analysis, the modified nucleobases can be analyzed by treating with an alkaline aqueous solution or with heating to dissociate the target DNA captured on the solid phase from the solid phase for obtaining a solution containing DNA fragments, and subjecting this solution to the nanopore analysis. In the present invention, a modification frequency of the target DNA by the modified nucleobases may be evaluated by taking into account an amount of the genomic DNA or the target DNA to be used.

Hereinafter, the present invention will be described with reference to Examples, but the present invention is not limited to these Examples.

### Reference Example 1: Evaluation of uncleaved ratio after genomic DNA cleavage reaction with restriction enzyme (1)

### (1-1) Cleavage of genomic DNA

1.8 µg of human genomic DNA (supplied from Clontech) and 3 units of the restriction enzyme XspI (suppled from Takara Bio) were dissolved in 20 µL of reaction buffer (20 mM Tris-HCl (pH=8.5), 10 mM MgCl₂, 1 mM DTT, 100 mM KCl), and the mixture was reacted at 37°C for 0, 20, 40 or 60 minutes to obtain genomic DNA cleaved by the restriction enzyme XspI.

### (1-2) Evaluation of uncleaved ratio of genomic DNA

A ratio of fragments cleaved by the restriction enzyme XspI was measured by amplifying the genomic DNA obtained above and cleaved by the restriction enzyme by real-time PCR using a primer set designed to correspond to a region that covers both sides of a cleavage site of the restriction enzyme XspI.

Conditions for the real-time PCR are shown below.

| | |
|---|---|
| Premix PCR reagent (KOD SYBR qPCR Mix: supplied from TOYOBO): | 12.5 µL |
| Forward primer (10 µM): | 0.5 µL |
| Reverse primer (10 µM): | 0.5 µL |
| 50× ROX reference dye: | 0.05 µL |
| Genomic DNA cleaved with restriction enzyme and diluted to 25 times: | 2 µL |
| Total | 25 µL |

The sequence of the forward primer is 5'-TCT AGA CCC CGC CCC ACG-3' (SEQ ID NO:1) and the sequence of the reverse primer is 5'-CTG CAG GAC CAC TCG AGG CTG-3' (SEQ ID NO:2), which were artificially synthesized and supplied by Hokkaido System Science.

An amplification reaction was performed using the above reaction solution having the above composition according to the following protocol:
(1) 98°C for 2 minutes
(2) 98°C for 10 seconds
(3) 68°C for 1 minute

First, a reaction step (1) was performed, and then reaction steps (2) and (3) were repeated in 50 cycles. Results are shown in Table 1.

**Table 1. Uncleaved ratio after genomic DNA cleavage reaction with restriction enzyme for various reaction times.**

| Treatment time with restriction enzyme | DNA amount(ng) | Uncleaved ratio |
|---|---|---|
| 0 minute | 1304 | - |
| 20 minutes | 811 | 44.9% |
| 40 minutes | 548 | 30.1% |
| 60 minutes | 474 | 25.7% |

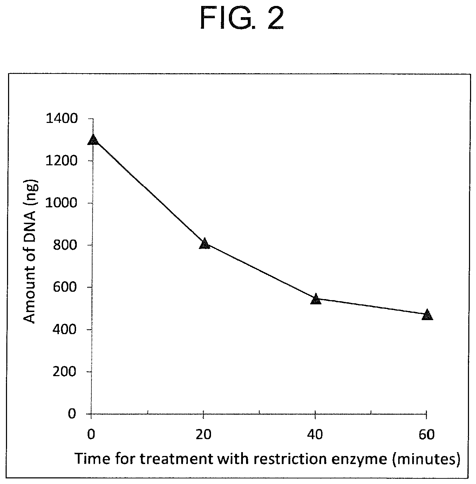

As a result, even when the genomic DNA was cleaved with the restriction enzyme XspI, it was confirmed that the reaction time of 60 minutes was not enough to allow the genomic DNA to be cleaved completely (Table 1, Fig. 2).

### Reference Example 2: Evaluation of uncleaved ratio after genomic DNA cleavage reaction with restriction enzyme (2)

### (2-1) Cleavage of genomic DNA

1.8 µg of human genomic DNA (supplied from Clontech) and 3 units of the restriction enzyme XspI (suppled from Takara Bio) were dissolved in 20 µL of reaction buffer (20 mM Tris-HCl (pH=8.5), 10 mM MgCl₂, 1 mM DTT, 100 mM KC1), and the mixture was reacted at 37°C for 20 minutes to obtain genomic DNA cleaved by the restriction enzyme XspI.

### (2-2) Evaluation of uncleaved ratio of DNA captured by nucleic acid probe specific for target DNA

The nucleotide sequence of a probe nucleic acid for capturing the target DNA (nucleic acid probe specific for the target DNA) is 5'-UGC AGG ACC ACU CGA GGC UGC CAC-3' (SEQ ID NO:3, a backbone of the nucleic acid is 2'-O-methylated RNA, and a 5'-terminus is labeled with biotin), which was artificially synthesized by Hokkaido System Science.

10 µL of genomic DNA obtained above and cleaved with the restriction enzyme XspI was dissolved in 90 µL of buffer (100 mM Tris-Cl, 1.5 M imidazole, 50 mM EDTA·2Na) containing the nucleic acid probe specific for the target DNA (1 pmol). The mixture was reacted at 95°C for 5 minutes and subsequently reacted at 37°C for one hour to form a hybrid of the target DNA in the genomic DNA and the nucleic acid probe specific for the target DNA. To a solution after the hybridization reaction, 50 µL of magnetic particles coated with 375 µg/mL of streptavidin (supplied from JSR, Magnosphere MS300/Streptavidin) was added and reacted at 37°C for 30 minutes to immobilize the hybrid of the nucleic acids onto the magnetic particles. These magnetic particles were washed twice with 250 µL of TBS-T, and suspended in 20 µL of distilled water. Subsequently, an amount of DNA captured on the magnetic particles and an amount of DNA not cleaved with the restriction enzyme XspI were measured by real-time PCR amplification using a primer set designed to correspond to a region that covers both sides of a cleavage site of the restriction enzyme XspI and a primer set designed to correspond to a region that does not cover both sides of the cleavage site.

Conditions for the real-time PCR amplification are shown below.

| | |
|---|---|
| Premix PCR reagent (KOD SYBR qPCR Mix: supplied from TOYOBO): | 12.5 µL |
| Forward primer (10 µM): | 0.5 µL |
| Reverse primer (10 µM): | 0.5 µL |
| 50× ROX reference dye: | 0.05 µL |
| DNA sample captured on magnetic particles | 2 µL |
| Total | 25 µL |

In the primer set designed to correspond to the region that covers both sides of the cleavage site of the restriction enzyme XspI, the sequence of the forward primer is 5'-TCT AGA CCC CGC CCC ACG-3' (SEQ ID NO:1) and the sequence of the reverse primer is 5'-CTG CAG GAC CAC TCG AGG CTG-3' (SEQ ID NO:2), which were artificially synthesized by Hokkaido System Science.

In the primer set designed to correspond to the region that does not cover both sides of the cleavage site, the sequence of the forward primer is 5'-TAG AAC GCT TTG CGT CCC GAC-3' (SEQ ID NO:4) and the sequence of the reverse primer is 5'-GAG AGC TCC GCA CTC TTC C-3' (SEQ ID NO:5), which were artificially synthesized by Hokkaido System Science.

An amplification reaction was performed using the above reaction solution of the above composition according to the following protocol:
(1) 98°C for 2 minutes
(2) 98°C for 10 seconds
(3) 68°C for 1 minute

First, the reaction step (1) was performed, and subsequently the reaction steps (2) and (3) were repeated in 50 cycles. Results are shown in Table 2.

**Table 2. Evaluation of uncleaved ratio of genomic DNA captured by nucleic acid probe specific for target DNA**

| | amount of DNA (ng) | Uncleaved ratio |
|---|---|---|
| Amount of captured DNA* | 296.1 | 3.9% |
| Amount of DNA not cleaved with restriction enzyme** | 11.7 | |

| | | |
|---|---|---|
| *Amount of both completely cleaved DNA fragments consisting of target DNA and incompletely cleaved DNA fragments comprising target DNA **Amount of incompletely cleaved DNA fragments comprising target DNA | | |

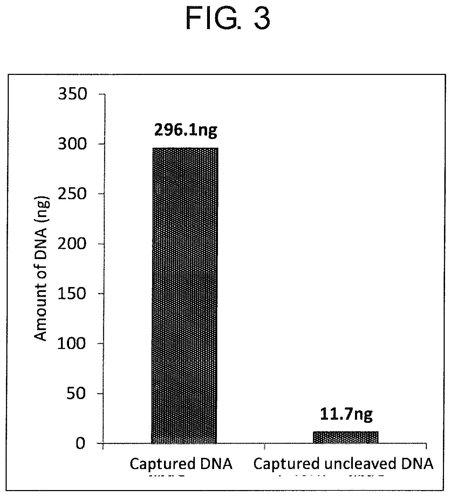

As a result, it was confirmed that DNA not cleaved with the restriction enzyme XspI occupied 3.9% of the target DNA captured onto the magnetic particles (Table 2, Fig. 3).

### Reference Example 3: Evaluation of removal efficiency of incompletely cleaved DNA fragments comprising target DNA using nucleic acid probe specific for non-target DNA

### (3-1) Cleavage of genomic DNA

1.8 µg of human genomic DNA (supplied from Clontech) and 3 units of the restriction enzyme XspI (suppled from Takara Bio) were dissolved in 20 µL of the reaction buffer (20 mM Tris-HCl (pH=8.5), 10 mM MgCl₂, 1 mM DTT, 100 mM KCl), and the mixture was reacted at 37°C for 20 minutes to obtain genomic DNA cleaved by the restriction enzyme XspI.

### (3-2) Removal of incompletely cleaved DNA fragment comprising target DNA

The nucleotide sequence of a probe nucleic acid for capturing the target DNA (nucleic acid probe specific for the target DNA) is 5'-UGC AGG ACC ACU CGA GGC UGC CAC-3' (SEQ ID NO:3, a backbone of the nucleic acid is 2'-O-methylated RNA, and a 5'-terminus is labeled with biotin), which was artificially synthesized by Hokkaido System Science. The nucleotide sequence of a probe nucleic acid for capturing the target DNA not cleaved with the restriction enzyme (nucleic acid probe specific for non-target DNA, having polyadenine) is 5'-GTG GGG CGG GGT CTA GAG AAA AAA AAA AAA AAA AAA AAA AAA AAA AAA-3' (SEQ ID NO:6), which was artificially synthesized by Hokkaido System Science.

10 µL of genomic DNA obtained above and treated with the restriction enzyme XspI was dissolved in 90 µL of buffer (100 mM Tris-Cl, 1.5 M imidazole, 50 mM EDTA·2Na) containing the nucleic acid probe specific for the target DNA (1 pmol), the nucleic acid probe specific for the non-target DNA (1 pmol), and magnetic particles (supplied from Invitrogen, Dynabeads Oligo (dT) 25; particle 1) coated with 37.5 µg of polythymine.

A solution having the same composition as above except that magnetic particles (supplied from Thermo Fisher, Sera-Mag Magnetic Oligo (dT) Microparticles; particle 2) were used in place of the particle 1 was prepared.

Further, a solution to which the nucleic acid probe specific for the non-target DNA was not added, and a solution to which the magnetic particles coated with polythymine were not added were prepared.

These solutions were first reacted at 95°C for 5 minutes, and subsequently reacted at 37°C for one hour to form a hybrid of the target DNA in the genomic DNA and the nucleic acid probe specific for the non-target DNA. Simultaneously, a hybrid of three, the incompletely cleaved DNA fragments comprising the target DNA, the nucleic acid probe specific for the non-target DNA, and the magnetic particles coated with polythymine was formed. Components in the solution after the hybridization reaction were magnetically collected on a magnet to precipitate incompletely cleaved DNA fragments comprising the target DNA in the solution, followed by separating them.

### (3-3) Evaluation of removal efficiency of incompletely cleaved DNA fragments comprising target DNA

To a supernatant of the solution obtained above (the incompletely cleaved DNA fragments comprising the target DNA were removed), 50 µL of magnetic particles coated with 375 µg/mL of streptavidin (supplied from JSR, Magnosphere MS300/Streptavidin) were added, and the mixture was reacted at 37°C for 30 minutes to immobilize the hybrid of the nucleic acids onto the magnetic particles. These magnetic particles were washed twice with 250 µL of TBS-T, and suspended in 20 µL of distilled water. Subsequently, an amount of DNA captured onto the magnetic particles and an amount of DNA not cleaved with the restriction enzyme XspI were measured by real-time PCR amplification using the primer set designed to correspond to the region that covers both sides of the cleavage site of the restriction enzyme XspI and the primer set designed to correspond to the region that does not cover both sides of the cleavage site.

Conditions for the real-time PCR amplification are shown below.

| | |
|---|---|
| Premix PCR reagent (KOD SYBR qPCR Mix: supplied from TOYOBO): | 12.5 µL |
| Forward primer (10 µM) : | 0.5 µL |
| Reverse primer (10 µM): | 0.5 µL |
| 50× ROX reference dye: | 0.05 µL |
| DNA sample captured onto magnetic particles | 2 µL |
| Total | 25 µL |

In the primer set designed to correspond to the region that covers both sides of the cleavage site of the restriction enzyme XspI, the nucleotide sequence of the forward primer is 5'-TCT AGA CCC CGC CCC ACG-3' (SEQ ID NO:1), and the sequence of the reverse primer is 5'-CTG CAG GAC CAC TCG AGG CTG-3' (SEQ ID NO:2), which were artificially synthesized by Hokkaido System Science.

In the primer set designed to correspond to the region that does not cover both sides of the cleavage site, the nucleotide sequence of the forward primer is 5'-TAG AAC GCT TTG CGT CCC GAC-3' (SEQ ID NO:4), and the sequence of the reverse primer is 5'-GAG AGC TCC GCA CTC TTC C-3' (SEQ ID NO:6), which were artificially synthesized by Hokkaido System Science.

An amplification reaction was performed using the above reaction solution having the above composition according to the following protocol.
(1) 98°C for 2 minutes
(2) 98°C for 10 seconds
(3) 68°C for 1 minute

First, the reaction step (1) was performed, and subsequently the reaction steps (2) and (3) were repeated in 50 cycles. Results are shown in Table 3.

**Table 3. Evaluation of removal efficiency of incompletely cleaved DNA fragments comprising target DNA using nucleic acid probe specific for non-target DNA**

| | Poly T particle | Captured DNA | Amount of DNA (ng) | Uncleaved ratio |
|---|---|---|---|---|
| Conventional method | | Total amount of DNA* | 240.4 | 3.1% |
| | | Amount of DNA uncleaved with restriction enzyme** | 7.4 | |
| Present Invention | Particle 1 | Total amount of DNA* | 268.0 | 1.3% |
| | | Amount of DNA uncleaved with restriction enzyme ** | 3.4 | |
| | Particle 2 | Total amount of DNA* | 246.8 | 0.7% |
| | | Amount of DNA uncleaved with restriction enzyme ** | 1.6 | |

| | | | | |
|---|---|---|---|---|
| *Amount of both completely cleaved DNA fragment consisting of target DNA and incompletely cleaved DNA fragments comprising target DNA **Amount of incompletely cleaved DNA fragments comprising target DNA | | | | |

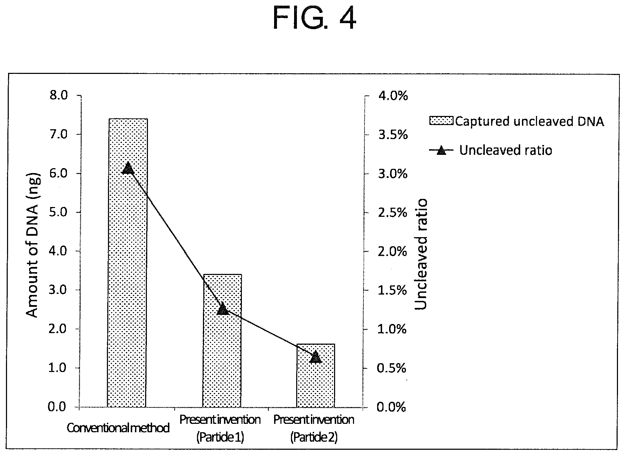

As a result, it was demonstrated that the incompletely cleaved DNA fragments comprising the target DNA could efficiently be removed by using the nucleic acid probe specific for the non-target DNA (Table 3, Fig. 4). Therefore, it is conceivable that the completely cleaved DNA fragment consisting of the target DNA can specifically be captured by the present methodology.

### Example 1: Analysis of methylcytosine in target DNA by immunoassay

### (1) Cleavage of genomic DNA

Genomic DNA is cleaved with a restriction enzyme in a solution to obtain a mixed solution containing the following DNA fragments (e.g., Fig. 1):
(a) completely cleaved DNA fragments consisting of target DNA;
(b) incompletely cleaved DNA fragments comprising target DNA; and
(c) various DNA fragments not comprising target DNA.

### (2) Removal of DNA fragment of (b)

(2-1) A 5'-flanking DNA probe and/or a 3'-flanking DNA probe (immobilized to magnetic particles) as nucleic acid probes specific for non-target DNA, and 2'-O-methylated RNA probe (5'-terminus is labeled with biotin) as a nucleic acid probe specific for target DNA are added to the mixed solution.
(2-2) The mixed solution is incubated to form a hybridization complex.
(2-3) The magnetic particles in the mixed solution were magnetically collected, and a supernatant (not containing the magnetic particles) of the mixed solution is collected. This supernatant is used as a solution in which an amount of the DNA fragments of (b) above is reduced and the DNA fragments of (a) and (c) above are contained. In this solution, the DNA fragment of (a) above forms the hybridization complex with 2'-O-methylated RNA probe.

### (3) Analysis of target DNA

(3-1) The solution obtained in (2) above is transferred to a multiwell plate to which streptavidin having affinity to biotin is immobilized. By so doing, the hybridization complex comprising the DNA fragment of (a) above and the 2'-O-methylated RNA probe is immobilized to the multiwell plate. Thus, the target DNA can be captured onto the solid phase.
(3-2) Methylcytosine in the target DNA captured onto the solid phase is analyzed by immunoassay (e.g., WO2015/025862; WO2015/025863; WO2015/025864; WO2015/108177; DNA Research 13, 37-42 (2006)).

### Example 2: Analysis of methylcytosine in target DNA by mass spectrometry, electrochemical analysis, or high performance liquid chromatography analysis

### (1) Cleavage of genomic DNA

Genomic DNA is cleaved by the same method as in Example 1(1).

### (2) Removal of DNA fragments of (b) above

The DNA fragments of (b) above are removed by the same method as in Example 1(2).

### (3) Analysis of target DNA

(3-1) The target DNA is treated by the same method as in Example 1(3-1).
(3-2) The target DNA captured to the solid phase is decomposed by endonuclease or/and exonuclease. By so doing, a solution in which the DNA fragment of (a) above is decomposed to monomer bases (nucleotides) can be collected.
(3-3) Methylcytosine in the target DNA decomposed to the monomer bases is analyzed by mass spectrometry (e.g., Analytical Chemistry 77(2), 504-510 (2005)), electrochemical analysis (e.g., Analytical Chemistry 83, 7595-7599 (2011)), or high performance liquid chromatography analysis (e.g., Nucleic Acids Research 34(8), e61 (2006)).

### Example 3: Analysis of methylcytosine in target DNA by nanopore analysis

### (1) Cleavage of genomic DNA

Genomic DNA is cleaved by the same method as in Example 1(1).

### (2) Removal of DNA fragments of (b)

The DNA fragments of (b) are removed by the same method as in Example 1(2).

### (3) Analysis of target DNA

(3-1) The target DNA is treated by the same method as in Example 1(3-1).
(3-2) A solution containing the DNA fragment of (a) above can be obtained by treating the target DNA captured onto the solid phase with an alkaline aqueous solution (e.g., 50 mM NaOH aqueous solution) or heating (e.g., 95°C for 2 minutes) to dissociate DNA from the solid phase.
(3-3) Methylcytosine contained in the target DNA dissociated from the solid phase is analyzed by nanopore analysis (e.g., Scientific Reports 501(2), srep00501 (2012)) .

### INDUSTRIAL APPLICABILITY

The method of the present invention is useful for measuring a target DNA.

## Claims

1. A method for measuring a target DNA, including
(1) cleaving a genomic DNA with a restriction enzyme in a solution to obtain a mixed solution containing (a) a completely cleaved DNA fragment consisting of the target DNA, (b) incompletely cleaved DNA fragments comprising the target DNA, and (c) DNA fragments not comprising the target DNA;
(2) removing (b) the incompletely cleaved DNA fragments comprising the target DNA from said mixed solution to obtain a solution containing (a) the completely cleaved DNA fragment consisting of the target DNA and (c) DNA fragments not comprising the target DNA; and
(3) analyzing the target DNA in said solution obtained in (2) .

2. The method according to claim 1 wherein said removal is performed using a nucleic acid probe specific for non-target DNA.

3. The method according to claim 2 wherein the nucleic acid probe specific for the non-target DNA is a following nucleic acid probe:
(1) a nucleic acid probe specific for 5'-flanking DNA;
(2) a nucleic acid probe specific for 3'-flanking DNA; or
(3) a combination of the nucleic acid probe specific for the 5'-flanking DNA and the nucleic acid probe specific for the 3'-flanking DNA.

4. The method according to any one of claims 1 to 3 wherein said analysis is performed using a nucleic acid probe specific for the target DNA.

5. The method according to any one of claims 1 to 4 wherein a modified nucleobase in the target DNA is analyzed.

6. The method according to claim 5 wherein the modified nucleobase is methylcytosine.

7. The method according to any one of claims 1 to 6 wherein said analysis is performed by immunoassay, mass spectrometry, electrochemical analysis, high performance liquid chromatography analysis, or nanopore analysis.
